(19) Europäisches Patentamt / European Patent Office / Office européen des brevets

(11) **EP 3 871 682 A1**

(12) **EUROPEAN PATENT APPLICATION**

(43) Date of publication:
01.09.2021 Bulletin 2021/35

(51) Int Cl.:
*A61K 31/728* (2006.01)  *A61P 27/04* (2006.01)

(21) Application number: 21167346.2

(22) Date of filing: 02.10.2017

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 14.10.2016 US 201662408559 P
08.06.2017 US 201762516911 P

(62) Document number(s) of the earlier application(s) in accordance with Art. 76 EPC:
**17817868.7 / 3 525 799**

(71) Applicant: **i.com Medical GmbH**
**81241 München (DE)**

(72) Inventors:
• MULLER-LIERHEIM, Wolfgang, Georg, Konrad
81243 München (DE)
• VAN SETTEN, Gysbert-Botho
182 33 Danderyd (SE)

(74) Representative: **Gill Jennings & Every LLP**
**The Broadgate Tower**
**20 Primrose Street**
**London EC2A 2ES (GB)**

Remarks:
This application was filed on 08-04-2021 as a divisional application to the application mentioned under INID code 62.

(54) **METHOD FOR ESTABLISHING, RESTORING, AND PRESERVING HOMEOSTASIS OF THE OCULAR SURFACE**

(57) The invention concerns a method for reducing or delaying the onset of irritation, discomfort, inflammation (acute or chronic), and/or an immune response at the ocular surface by topically administering a fluid comprising high molecular weight hyaluronic acid to the ocular surface of the eye of a human or non-human animal subject, wherein the hyaluronic acid has an intrinsic viscosity of > 2.5 $m^3$/kg and a concentration of < 0.2 % w/v.

**Description**

CROSS-REFERENCE TO RELATED APPLICATIONS

The present application claims the benefit of U.S. Provisional Application Serial No. 62/516,911, filed June 8, 2017, and U.S. Provisional Application Serial No. 62/408,559, filed October 14, 2016, each of which is hereby incorporated by reference herein in its entirety, including any figures, tables, nucleic acid sequences, amino acid sequences, or drawings.

FIELD OF THE INVENTION

The present invention concerns a fluid for reducing or delaying the onset of irritation, discomfort, inflammation, and/or an immune response at the ocular surface, and/or after onset, its attenuation and long-term alleviation.

BACKGROUND OF THE INVENTION

Dry eye disease is a common ocular condition with significant impact on vision and quality of life. The term dry eye gained recognition in the beginning of the past century, being primarily used in relation to patients with Sjogren disease. Over decades, the diagnosis of dry eye disease relied mainly on staining of the cornea and the conjunctiva, estimation of tear break-up time and the Schirmer test. The currently accepted number one therapy for dry eye is some form of tear substitute for the amelioration of symptoms.

In 2007, the Dry Eye Workshop (DEWS) published the consensus of a group of about 50 recognized international experts on the current knowledge of dry eye disease. This consensus paper has become the global reference for clinical research on dry eye disease. The concept of aqueous deficient versus hyper-evaporative dry eye, the role of tear osmolarity as a key triggering factor for ocular inflammation, and the focus on Meibomian gland dysfunction are among the outcomes of the DEWS. The lack of predictiveness of signs assessed by individual diagnostic test methods for the symptoms experienced by the patient is known as disassociation between signs and symptoms, and is reflected in the published literature. New test methods have become available such as determination of tear osmolarity and visualization of the Meibomian glands.

More recently, the clinical dry eye research concentrates on inflammatory processes at the cellular and molecular level. Impression cytology with count of Goblet cells and flow-cytometry for markers like HLA-DR, as well as testing for MMP-9 level are results of this focus. The development of new therapies involves investigating anti-inflammatory compounds that are promising to interrupt the chronically self-maintained cycle of inflammation. Candidates include cyclosporine, corticosteroids, doxycycline and essential fatty acids. The therapeutic objective is to regain ocular surface immune homeostasis. In this respect, the current clinical research on dry eye disease touches other ocular immune responses such as allergic keratoconjunctivitis.

BRIEF SUMMARY OF THE INVENTION

The present invention provides a holistic approach for establishing, restoring, and preserving the homeostasis of an ocular surface. By topically administering a fluid composition containing a very high molecular weight form of hyaluronic acid (also called hyaluronan or HA) to the ocular surface, the invention can reduce or delay the onset of irritation, discomfort (such as itchiness or ocular pain), inflammation (acute or chronic), and/or an immune response, at an ocular surface.

DETAILED DESCRIPTION OF THE INVENTION

Hyaluronic acid (HA) is a carbohydrate - a mucopolysaccharide, specifically, which can be found in living organisms. The biological functions of endogenous HA include maintenance of the elastoviscosity of liquid connective tissues such as joint synovial fluid and eye vitreous fluid (Necas J et al., "Hyaluronic acid (hyaluronan): a review", Veterinarni Medicina, 2008, 53(8):397-411; Stern R et al., "Hyaluronan fragments: An information-rich system", European Journal of Cell Biology, 2006, 85:699-715). Although the specific mechanisms involved in the diverse signaling of HA are still poorly understood, it is known that HA can modulate multi-faceted biological effects that can vary depending on HA size (Cyphert JM et al., "Size Matters: Molecular Weight Specificity of Hyaluronan Effects in Cell Biology," International Journal of Cell Biology, 2015, Epub 2015 Sep 10, 563818).

Sodium hyaluronate and other viscoelastic agents have been used in intraocular surgery since the 1970s and for treatment of dry eyes since the 1980s (Higashide T and K Sugiyama, "Use of viscoelastic substance in ophthalmic surgery- focus on sodium hyaluronate," Clinical Ophthalmology, 2008, 2(1):21-30; Polack FM and MT McNiece, "The treatment of dry eyes with Na hyaluronate (Healon) - preliminary report, 1982, 1(2): 133-136); however, little attention has been paid thus far to the biological function of hyaluronic acid in epithelia (Muller-Lierheim WGK, "Tranenersatzlosungen, Neues über Hyaluronsaure," Aktuelle Kontaktologie, April 2015, 17-19).

An aspect of the invention includes a method for reducing or delaying the onset of a condition selected from among an irritation, discomfort (*e.g.,* itchiness or ocular pain), inflammation, immune response, or combination of two or more of the foregoing, at an ocular surface, comprising topically administering a fluid comprising high molecular weight hyaluronic acid (HA) to the ocular surface of the eye of a human or non-human animal subject. The hyaluronic acid is high molecular weight, having an intrinsic viscosity of greater than 2.5 $m^3/kg$, and a concentration of < 0.2 % w/v. The intrinsic viscosity may be determined by the method of the European Pharmacopoeia 9.0, "Sodium Hyaluronate", page 3584 (which is incorporated herein by reference in its entirety). Briefly, the intrinsic viscosity $[\eta]$ is calculated by linear least-squares regression analysis using the Martin equation: $\mathrm{Log}_{10} (n_r\text{-}1/c) = \log_{10} [\eta] + \kappa[\eta]c$. In some embodiments, the hyaluronic acid has an intrinsic viscosity of greater than 2.9 $m^3/kg$.

In some embodiments, the HA has a molecular weight of at least 3 million Daltons as calculated by the Mark-Houwink equation. In some embodiments, the HA has a molecular weight in the range of 3 million to 4 million Daltons as calculated by the Mark-Houwink equation.

In some embodiments, the high molecular weight HA is hyaluronan. In some embodiments, the high molecular weight HA is cross-linked, such as hylan A and hylan B. In some embodiments, the high molecular weight HA is non-cross-linked. In some embodiments, the high molecular weight HA is linear. In some embodiments, the high molecular weight HA is non-linear. In some embodiments, the high molecular weight HA is a derivative of hyaluronan, such as an ester derivative, amide derivative, or sulfated derivative, or a combination of two or more of the foregoing.

In some embodiments, the ocular surface to which the fluid is administered is in homeostasis at the time the fluid is administered. In other embodiments, the ocular surface of the eye of the subject is not in homeostasis.

The administered fluid can increase or enhance the visual performance of the eye to which it is administered, whether or not the ocular surface is in homeostasis at the time the fluid is administered to the eye. The administered fluid helps to stabilize the fluid film of the eye (*e.g.,* the tear film at the ocular surface), optimizing vision and visual performance, which is particularly beneficial in extreme conditions such as aviation and other settings in which it is impossible or undesirable to blink, space travel, diving in polluted or intoxicated fluids, or operating in areas with extreme climates such as cold, heat, and dryness.

An increase or enhancement of visual performance can be defined as an increase or enhancement in the speed and/or accuracy of processing visual information. For example, visual performance can be described as how quickly and accurately an individual can process visual stimuli that are defined in terms of criteria such as adaptation luminance, target contrast, and target size. Methods for assessing visual performance and changes in visual performance are known in the art (see, for example, Toda I et al., "Visual performance after reduced blinking in eyes with soft contact lenses or after LASIK," J Refract. Surg., 2009, Jan., 25(1):69-73; and Rea MS and MJ Quellette, "Relative visual performance: A basis for application," Lighting Res. Technol., 1991, 23(3):135-144, which are incorporated herein by reference in their entirety).

The fluid may be administered to the ocular surface of one or both eyes of the subject by any topical administration method. For example, the fluid may be administered as one or more drops from a device for dispensing eye drops, such as an eye dropper. The fluid may be self-administered or administered by a third party. The dosage administered, as single or multiple doses, to an ocular surface will vary depending upon a variety of factors, including patient conditions and characteristics, extent of symptoms, concurrent treatments, frequency of treatment and the effect desired. For example, one or more drops (of, for example, about 30 microliters each) may be administered. Typically, administration of 1-3 drops, one to three times per day, will be sufficient, particularly for acute ocular surface inflammation conditions. In cases of chronic ocular surface inflammation, however, more frequent administration may be needed, particularly during the initial phase of treatment, *e.g.,* 1-3 drops for four, five, six, seven, eight, nine, ten, or more times per day.

Advantageously, in some embodiments, the frequency of administration and/or the amounts per dose can be decreased with time, as homeostasis of the ocular surface is preserved or restored and the cornea is stabilized. For example, in some cases, after four weeks, the amount administered may be reduced and/or the frequency of administrations each day may be reduced or the frequency of administrations may be reduced to semi-daily.

The fluid may be administered prophylactically, before the condition exists, *i.e.,* before the irritation, discomfort (*e.g.,* ocular pain), inflammation, or immune response develops, to reduce the severity of the condition and/or delay its onset; or the fluid may be administered therapeutically, after the condition exists, to reduce the severity of the condition. Optionally, the fluid is administered prophylactically before an event or stimulus occurs that causes the condition, such as ocular trauma (*e.g.,* non-surgical trauma), ocular surgery (*e.g.,* glaucoma surgery, squint surgery, *etc.*), or infection of the eye. In some embodiments, onset of the condition is delayed indefinitely (*i.e.,* prevented). In some embodiments, one or more symptoms of the condition are alleviated or eliminated. In some embodiments, all symptoms of the condition are alleviated or eliminated.

The fluid may also be administered prophylactically to subjects that are particularly susceptible or prone to infection, in order to prevent or delay onset of irritation, inflammation, immune response, or a combination of two or more of foregoing. For example, the subject may be immunocompromised. The subject's immunocompromised condition may have one or more causes, such as a medical treatment (*e.g.,* radiation therapy, chemotherapy or other immunosuppressing treatment), environmental exposure (*e.g.,* radiation exposure), or genetic defect.

In cases in which the condition exists at the time of fluid administration and the fluid is administered therapeutically, optionally, the method further comprises the step of identifying the subject as having the condition prior to administering the fluid.

In some embodiments, the condition may have one or more of the following characteristics: leukocyte invasion at the ocular surface and tears, CD44 upregulation at the ocular surface, and activation of an immune cascade that includes one or more of IL-1, IL-2, IL-5, IL-6, IL-8, CXCL8, IL-10, IL-12, IL-16, IL-33, MCP1, CCL2, MIP1d (also known as CCL15), ENA-78, CXCL5, SILR1, sIL-6R, sgp sEGFR, sTNFR, I-17A, IL-21, IL-22, CXCL9, MIG, CXCL11, I-TAC, CXCL10, IP-10, MIP-1β, CCL4, RANTES, and CCL5.

The condition may be caused by various stimuli - external, internal, or both. In some embodiments, the condition is caused by an external stimulus resulting in a disruption of the smoothness and/or integrity of the ocular surface (*e.g.,* medical therapy, ocular surgery, non-surgical trauma, contact lens wearing, microbial infection, allergen, hapten, toxic agent, or irritative substance).

Various medical therapies, such as small molecule pharmaceuticals, and biologics, may cause the condition. For example, the condition may be caused by a "beta blocker", which refers to agents that inhibit or block the activity of one or more beta-adrenergic receptors. Beta blockers may be used for treatment of hypertension, stable and unstable angina, arrhythmias, migraine, bleeding esophageal varices, heart failure, and coronary artery disease, among other indications. Some beta-blockers antagonize one specific subtype of beta-adrenergic receptors (*e.g.,* a beta-1 selective beta blocker which selectively antagonizes the beta-1 adrenergic receptor), whereas other beta-blockers are non-selective. Some beta-blockers can inhibit the effect of ligands such as noradrenaline or norepinephrine on one or more beta-adrenergic receptors. Accordingly, the term "beta-blocker" refers to all types of antagonists or inhibitors of beta-adrenergic receptors, regardless of whether the beta-blocker antagonizes one, two or more beta-adrenergic receptors and regardless of whether they affect other processes. Examples of beta-blockers include, but are not limited to, acebutolol, alprenolol, atenolol, betaxolol, bisoprolol, bopindolol, bucindolol, butaxamine, carteolol, carvedilol, celiprolol, esmolol, labetalol, levobunolol, medroxalol, metipranolol; metoprolol, nadolol, nebivolol, nadolol, oxprenolol, penbutolol, pindolol, propafenone, propranolol, sotalol, timolol, and eucommia bark.

In some embodiments, the condition is allergy of the eye. In some embodiments, the condition is non-infectious keratoconjunctivitis caused by an external damage, allergic keratoconjunctivitis (such as seasonal allergic keratoconjunctivitis), or infectious keratoconjunctivitis such as viral keratoconjunctivitis, bacterial conjunctivitis, fungal keratoconjunctivitis, and parasitic conjunctivitis. In some embodiments, the condition is caused by an internal stimulus (*e.g.,* hormonal disturbance (such as menopause and andropause), rheumatic disease, epithelial-mesenchymal transition (EMT), or autoimmune disease).

The condition may be caused by a wound of the eye. In some embodiments, the wound is caused by physical trauma, chemical trauma, or radiation (radiation injury). In some embodiments, the wound is caused by an ocular surgery. Examples of ocular surgeries include but are not limited to natural or artificial corneal transplantation, corneal implantation (*e.g.,* intracorneal rings (ICRs), and keratoprosthesis), glaucoma surgery, cataract surgery (*e.g.,* phacoemulsification, extrapsular cataract surgery, or intracapsular surgery), refractive surgery (*e.g.,* radial keratotomy or refractive corneal incision), retinal surgery, squint (strabismus) surgery, corrective laser eye surgery (*e.g.,* laser-assisted *in situ* keratomileusis (LASIK) or photorefractive keratectomy (PRK)), and cross-linking surgery. Administration of the fluid of the invention before, during, and/or after ocular surgery, such as glaucoma surgery, can improve clinical outcome, for example, by accelerating recovery, including recovery of visual performance after surgery, reducing scarring, and reducing itching, irritation, pain, and other discomfort.

The fluid may be administered to reduce or prevent or delay onset of ocular discomfort such as itchiness or ocular pain. The pain may have one or more causes. For example, the ocular pain may be pain associated with mechanical, chemical, or thermal stimulation of the ocular surface. The ocular pain may be associated with acute or chronic inflammation, or immune response. With the reduction of the pain, comes the reduction of secondary neuroinflammatory effects (Belmonte C et al., "TFOS DEWS II pain and sensation report", The Ocular Surface, 15:404-437). The cause of the pain may be known or unknown.

Optionally, the fluid further includes one or more bioactive agents (*e.g.,* a hydrophobic active ingredient). As used herein, the term "bioactive agent" refers to any substance that has an effect on the human or non-human animal subject when administered in an effective amount to affect the tissue. The bioactive agent may be any class of substance such as a drug molecule or biologic (*e.g.,* polypeptide, carbohydrate, glycoprotein, immunoglobulin, nucleic acid), may be natural products or artificially produced, and may act by any mechanism such as pharmacological, immunological, or metabolic. Examples of classes of bioactive agents include substances that modify the pressure of the eye (*e.g.,* enzyme inhibitors) and anti-angiogenic agents. Some specific examples of bioactive agents include steroids (*e.g.,* corticosteroids), antibiotics, tacrolimus, plasmin activator, anti-plasmin, and cyclosporin A. In some embodiments, the bioactive agent is a steroid or antibiotic to treat or prevent eye infection; glaucoma drug such as prostaglandin analog, beta blocker, alpha agonist, or carbonic anhydrase inhibitor; agent for allergy eye relief such as histamine antagonist or non-steroidal anti-inflammatory drug; or mydriatic agent. Unfortunately, in some cases, the bioactive agent or agents included in the fluid may be irritative or damaging to the eye (*e.g.,* cyclosporin A). Advantageously, through its rheological property and other properties, the high molecular weight HA in the fluid can alleviate and/or protect the eye from the irritative and/or damaging effects of the biologically active agent or agents within the fluid (*i.e.,* the bioactive agent would be more irritative or more damaging to the eye if administered without the high molecular weight HA).

In some embodiments, the fluid contains no steroid, antibiotic, antibiotic, or immunomodulator. In some embodiments, the fluid contains no other bioactive agent (*e.g.,* no hydrophobic active ingredient).

In some circumstances, it may be desirable to include one or more preservatives or detergents within the fluid. Often, such preservatives and detergents are irritative or damaging to the eye. Advantageously, through its rheological property and other properties, the fluid can alleviate and/or protect the eye from the irritative and/or damaging effects of the preservative or detergent within the fluid. Thus, in some embodiments, the fluid further comprises a preservative or detergent that is irritative or damaging to the eye (*i.e.,* a preservative or detergent that would be more irritative or more damaging to the eye if administered without the high molecular weight HA). In some embodiments, the fluid contains no preservative or detergent.

In some embodiments, the fluid includes cyclosporin A, cetalkoniumchloride, tyloxapol, or a combination of two or more of the foregoing.

In some embodiments, the fluid is administered to the subject before, during, and/or after administration of another composition comprising a bioactive agent to the subject. In some circumstances, it may be desirable to include one or more preservatives or detergents within the other composition. As indicated above, often, such preservatives and detergents are irritative or damaging to the eye, and some bioactive agents themselves may be irritative or damaging to the eye. Advantageously, through its rheological property and other properties, the fluid can alleviate and/or protect the eye from the irritative and/or damaging effects of the bioactive agent, preservative, and/or detergent within the other composition. Thus, the bioactive agent, preservative, and/or detergent within the other composition would be more irritative or more damaging to the eye if administered without the fluid.

In some embodiments, the other composition includes cyclosporin A, cetalkoniumchloride, tyloxapol, or a combination of two or more of the foregoing.

The other composition administered to the subject may be in any form and administered by any route (*e.g.,* local or systemic). In some embodiments, the other composition is administered to the eye, e.g., topically or by injection. In some embodiments, the other composition is topically administered to the ocular surface.

In some embodiments, the preservative or detergent included in the fluid or other composition is a chemical preservative or oxidative preservative.

In some embodiments, the preservative or detergent included in the fluid or other composition is one that kills susceptible microbial cells by disrupting the lipid structure of the microbial cell membrane, thereby increasing microbial cell membrane permeability.

In some embodiments, the preservative or detergent included in the fluid or other composition is one that causes damage to the corneal tissues, such as corneal epithelium, endothelium, stroma, and interfaces such as membranes.

In some embodiments, the preservative or detergent included in the fluid or other composition is selected from the group consisting of quaternary ammonium preservative (e.g., benzalkonium chloride (BAK) or cetalkoniumchloride), chlorobutanol, edetate disodium (EDTA), polyquaternarium-1 (*e.g.,* Polyquad™ preservative), stabilized oxidizing agent (*e.g.,* stabilized oxychloro complex (*e.g.,* Purite™ preservative)), ionic-buffered preservative (*e.g.,* sofZia™ preservative), polyhexamethylene biguanide (PHMB), sodium perborate (*e.g.,* GenAqua™ preservative), tylopaxol, and sorbate.

In some embodiments, the fluid is at least essentially mucin-free; or in other words having a mucin concentration of < 0.3 % w/v.

In some embodiments, the fluid includes a preservative. In other embodiments, the fluid does not include a preservative (*i.e.,* the fluid is preservative-free).

In some embodiments, the fluid further includes a glycosaminoglycan (GAG), *i.e.,* includes one or more GAGs in addition to the high molecular weight HA; electrolyte (*e.g.,* sodium chloride); buffer (*e.g.,* phosphate buffer); or a combination of two or more of the foregoing.

The subject may or may not have dry eye syndrome (the aqueous tear deficiency type or qualitative dry eye type) at the time the fluid is administered to the eye of the subject. In some embodiments of the therapeutic or prophylactic methods, the condition is an irritation, discomfort, inflammation, immune response, or combination of two or more of the foregoing, at an ocular surface, and the eye of the subject to which the fluid is administered does not have aqueous tear deficiency (ATD) at the time of administering the fluid (*i.e.,* in the absence of ATD). In some embodiments of the therapeutic or prophylactic methods, the condition is an irritation, discomfort, inflammation, immune response, or combination of two or more of the foregoing, at an ocular surface, and the eye of the subject to which the fluid is administered does not have qualitative dry eye at the time of administering the fluid (*i.e.,* in the absence of qualitative dry eye). In some embodiments of the therapeutic or prophylactic methods, the condition is an irritation, discomfort, inflammation, immune response, or combination of two or more of the foregoing, at an ocular surface, and the eye of the subject to which the fluid is administered does not have dry eye syndrome at the time of administering the fluid (*i.e.,* in the absence of aqueous tear deficiency or qualitative dry eye).

In some embodiments of the therapeutic or prophylactic methods, the condition is an irritation, discomfort, inflammation, immune response, or combination of two or more of the foregoing, at an ocular surface, and the subject is not suffering from a tear volume deficiency; however, the subject has an ocular surface abnormality (a topographic anomaly) comprising elevations on the cornea or elsewhere on the eye surface for which the normal tear film (tear film of normal surface tension and viscosity) does not cover, resulting in areas of friction at the ocular surface.

The fluid may be used in conjunction with a bandage contact lens. Thus, the method may further include applying a bandage contact lens to the eye before, during, and/or after administering the fluid. For example, the fluid may be administered before applying the bandage contact lens, after the contact lens, and/or placing fluid on the bandage contact lens before applying the bandage contact lens to the eye. Use of the fluid allows the bandage contact lens to exert pressure on the ocular surface while simultaneously minimizing friction at the ocular surface. Advantageously, the fluid and bandage contact lens can safely be used shortly after ocular surgery, *e.g.,* glaucoma surgery.

Another aspect of the invention concerns a kit that may be used for carrying out the method of the invention described herein. The kit comprises the fluid described herein, and one or more bandage contact lenses. Bandage contact lenses may be packaged together with the fluid within the same container (with the bandage contact lenses in contact with the fluid), or the bandage contact lenses may be separate from the fluid, packaged in separate containers. Suitable containers include, for example, bottles, vials, syringes, blister pack, *etc.* The containers may be formed from a variety of materials such as glass or plastic.

The kit may include a delivery agent (separately or in assocation with the fluid) that is to be brought into contact with the ocular surface or other part of the eye. For example, the kit may include particles (*e.g.,* microparticles or nanoparticles) that are coated with the fluid and/or release the fluid onto the ocular surface.

Optionally, the kit may include a device for dispensing eye drops (*e.g.,* an eye dropper), which may or may not serve as a container for the fluid in the kit before the kit's outer packaging is accessed (*e.g.,* opened), *i.e.,* the eye drop dispensing device may function to contain the fluid provided in the unaccessed (unopened) kit, or may be empty and receive the fluid after the kit is accessed. Optionally, the kit may include a label or packaging insert with printed or digital instructions for use of the kit, *e.g.,* for carrying out the method of the invention.

Kits can include packaging material that is compartmentalized to receive one or more containers such as vials, tubes, and the like, each of the container(s) including one of the separate elements to be used in a method described herein. Packaging materials for use in packaging pharmaceutical products include, by way of example only U.S. Pat. Nos. 5,323,907, 5,052,558 and 5,033,252. Examples of pharmaceutical packaging materials include, but are not limited to, blister packs, bottles, tubes, pumps, bags, vials, light-tight sealed containers, syringes, bottles, and any packaging material suitable for a selected formulation and intended mode of administration and treatment.

A kit may include one or more additional containers, each with one or more of various materials desirable from a commercial and user standpoint for use of the compositions described herein. Non-limiting examples of such materials include, but not limited to, buffers, diluents, carrier, package, container, vial and/or tube labels listing contents and/or instructions for use, and package inserts with instructions for use.

A label can be on or associated with the container. A label can be on a container when letters, numbers or other characters forming the label are attached, molded or etched into the container itself; a label can be associated with a container when it is present within a receptacle or carrier that also holds the container, *e.g.,* as a package insert. A label can be used to indicate that the contents are to be used for a specific therapeutic application. The label can also indicate directions for use of the contents, such as in the methods described herein.

In some embodiments of the kit, the fluid can be presented in a pack or dispenser device which can contain one or more unit dosage forms containing a composition disclosed herein. The pack can for example contain metal or plastic foil, such as a blister pack. The pack or dispenser device can be accompanied by instructions for administration.

Acute and Chronic Ocular Surface Inflammation

Ocular inflammation may take the form of numerous eye disorders of varying severity depending on the location of the inflammation. Disorders attributed to ocular inflammation include, for example, uveitis, conjunctivitis, episcleritis, scleritis, optic neuritis, retrobulbar neuritis, keratitis, blepharitis, and the like. Many of these conditions can occur secondary to bacterial or viral infection. Ocular inflammation can also result following ophthalmologic surgical procedures or ocular trauma resulting from physical injury of the eye.

Inflammation is a normal component of the immune response and a necessary component of the healing process, but is detrimental if it proceeds at an inappropriate level or duration. An acute leucocytic inflammation is triggered within hours of exposure to the causative agent (e.g., antigen, wound to the eye, or other insult) and represents a normal, time-limited inflammatory reaction. The end of this phase varies, but reduced inflammation provides an indirect sign of improvement and slow reconstitution to a prior state.

In contrast to acute ocular inflammation, chronic ocular inflammation is a state that is not merely different quantitatively in duration (*i.e.*, prolonged), but also qualitatively different in kind. The differences between acute ocular inflammation and chronic ocular inflammation are significant, analogous to the differences between the immunological events in acute, healing wounds and chronic, non-healing wounds (see, for example, Tarnuzzer RW and Schultz GS, "Biochemical analysis of acute and chronic wound environments," Wound Repair Regen., 1996, Jul-Sep, 4(3):321-325). These differences in immunological events, particularly at conjunctival and subconjunctival tissue (as opposed to the cornea), underlie a diversity of ocular surface disorders and explain why some patients within a disease or disorder population are refractory to an otherwise effective treatment plan.

Ocular surface homeostasis is regulated by resident lymphocytes (CD8+ T cells, gamma delta T cells, and natural killer T cells) and CD4+ regulatory T cells, among other factors. These interact with anti-inflammatory factors, such as interleukin (IL)-1 receptor antagonist, transforming growth factor (TGF)-beta 2, and matrix protease inhibitors such as inhibitor of metalloproteinase (TIMP)-1. Stress factors including environment challenges, infections, endogenic stress, autoimmunity and genetic factors may all disturb the finely-tuned homeostatic balance existing on the ocular surface and activate an acute inflammatory response. The method of the invention provides a holistic approach to establishing, restoring, and preserving ocular surface homeostasis, and thereby reduce or delay the onset of irritation, discomfort, inflammation, immune response, or a combination of two or more of the foregoing, at the ocular surface. Thus, before after onset, administration of the fluid can result in attenuation and long-term alleviation of the irritation, discomfort, inflammation, immune response, or a combination of two or more of the foregoing, at the ocular surface.

Epithelial-derived pro-inflammatory cytokines activate immature resident antigen-presenting cells (APCs), which are mainly dendritic cells, on the ocular surface. Mature APCs migrate tot eh regional lymph nodes and initiate an adaptive immune response by priming naive CD4+ T cells including T helper (Th)1 and Th17 cells. Through activated angiogenesis and lymphangiogesis, these inflammatory mediators traffic back to the ocular surface, where Th1-secreted interferon (IFN)-gamma and Th17-secreted IL-17 increased cytokine production, induce epithelial and goblet cell apoptosis and alter conjunctival homeostasis, perpetuating a chronic inflammatory process. In a state of goblet cell deficiency, the deprivation of gel-forming mucins leads to increased friction, thus perpetuating inflammation (Pflugfelder SC et al., "Epithelial-immune cell interaction in dry eye," Cornea, 2008, 27 Suppl 1:S9-11).

Whereas acute ocular surface inflammation is characterized by a rapid onset and relatively shorter duration (typically, a few minutes to a number of days), protein exudate, and the cellular infiltrate of the conjunctival and subconjunctival tissue is mainly neutrophils, chronic ocular surface inflammation is characterized by a longer duration (a number of days to years), fewer neutrophils, the cellular infiltrate is mainly macrophages and lymphocytes, and proliferation of blood vessels, fibrosis and tissue necrosis. Indeed, the primary cells of chronic ocular surface inflammation are macrophages and lymphocytes at the conjunctiva and subconjunctiva.

Typically, chronic ocular surface inflammation can also be identified by ocular surface inflammation markers including one or more of: tear film hyperosmolarity; elevated matrix metalloproteinase-9 (MMP-9) expression by epithelial cells and infiltrating leucocytes at the ocular surface; increased MMP-9 activity at the ocular surface (its MMP-3 and TIMP-1-mediated enzymatic activity); elevated interferon-gamma; over-expression of human leukocyte antigen-antigen D related (HLA-DR) by conjunctival epithelial cells; and intracellular adhesion molecule 1 (ICAM-1) by conjunctival epithelial cells, increased number of activated lymphocytes, and decreased number of goblet cells.

Steroids (*e.g.*, corticosteroids) are considered the gold standard therapy for ocular inflammations. Dexamethasone, prednisolone, triamcinolone acetonide, fluocinolone acetonide and loteprednol etabonate are commonly employed, which act by various mechanisms such as inhibition of multiple inflammatory cytokines, fibrin deposition, polymorphonuclear leukocyte migration, and anti-angiogenesis effects by inhibition of nuclear factor-kappa-B (NF-kB) signal pathway (Nakao et al., Am J Pathol. 2007; 171(3): 1058-65).

The anti-inflammatory action of steroids is thought to be due to interference with arachidonic acid metabolism, *i.e.*, by inhibition of phospholipase A2 which causes the release of arachidonic acid from the tissue phospholipid pool. Although steroids are effective in the treatment of ocular inflammation, their extended use is complicated by severe side effects, the most common of which are cataract formation, glaucoma, and infectious eye diseases (Kymionis GD et al., "Treatment of chronic dry eye: treatment of cyclosporine," Clin Ophthalmol. 2008 Dec; 2(4): 829-836; Schultz C, "Safety and efficacy of cyclosporine in the treatment of chronic dry eye," Ophthalmol Eye Dis. 2014; 6: 37-42). Likewise, immunomodulatory drugs such as cyclosporine and anti-inflammatory agents such as tetracycline and its derivatives can cause ocular pain and irritation in some patients (*e.g.,* burning and itching), which can be difficult to tolerate in an inflamed eye.

Advantageously, topical administration of high molecular weight hyaluronic acid (HA), *e.g.,* 3-4 MDa, may be used as an effective natural immunomodulator to regain ocular surface immune homeostasis in patient groups with chronic ocular surface inflammation or seasonal allergic keratoconjunctivitis, without the aforementioned agents or their side effects. In particular, fluids of the invention may be used to restore immune homeostasis of the ocular surface in these patients.

The influence of the molecular weight of the HA molecules in eye drops on their bio-chemical function has very recently come into the focus of ophthalmic research. Due to its function in the extracellular matrix, HA plays an important role in the proliferation and differentiation of keratinocytes, in corneal wound healing, and in the homeostasis of the corneal epithelium. The prevalence of dry eyes in elderly persons is likely to be associated with the significant decrease of free HA in the extracellular matrix starting from the fifth decade. Moreover, HA acts as a signal molecule. Whereas, high molecular weight HA (3 - 4 MDa) acts anti-angiogenic and immunosuppressive, HA of medium molecular weight has inflammatory, immunostimulating, as well as angiogenic properties (Stern R et al., "Hyaluronan fragments: An information-rich system", European Journal of Cell Biology, 2006, 85:699-715; and Noble PW, "Hyaluronan and its catabolic products in tissue injury and repair," Matrix Biology, 2002, 21:25-29). Elevated enzyme levels in chronic inflammation cause increased hydrolysis of HA and thus a shift toward lower molecular weight. High molecular weight HA in eye drops can contribute to minimize this inflammation-related shift by decreasing friction at the ocular surface and supporting the down-regulation of the inflammatory process. A decrease of friction may also lead to a decrease of nociceptive neurological stimuli that can trigger the maintenance or onset of neurogenic inflammation supportive pathways.

It is well known that during acute and chronic inflammation various putative mediators of inflammation are released by the inflamed tissues and by leukocytes. The concentrations of these mediators and leukocytes are indicative of the level or degree of inflammation. Likewise, a reduction in concentration of these mediators and leukocytes is an indication of the effectiveness of a drug in treating inflammation. Examples of ocular surface inflammation markers include, but are not limited to, those listed above, i.e., tear film hyperosmolarity; elevated matrix metalloproteinase-9 (MMP-9) expression by epithelial cells and infiltrating leucocytes at the ocular surface; increased MMP-9 activity at the ocular surface (its MMP-3 and TIMP-1-mediated enzymatic activity); elevated interferon-gamma; over-expression of human leukocyte antigen-antigen D related (HLA-DR) by conjunctival epithelial cells; and intracellular adhesion molecule 1 (ICAM-1) by conjunctival epithelial cells, increased number of activated lymphocytes, and decreased number of goblet cells.

Optionally, before administration of the fluid of the invention, the method of the invention may further comprise identifying the subject/eye as having chronic ocular surface inflammation by detecting or measuring one or more of these inflammatory markers of the ocular surface. Optionally, the method may further comprise detecting or measuring one or more of these inflammatory markers of the ocular surface one or more times after administration of the fluid of the invention in order to monitor the inflammatory status of the eye(s) over time and guide the frequency and duration of further administration of the fluid of the invention.

Fluid Preparation

As indicated above, the hyaluronic acid of the fluid has an intrinsic viscosity of greater than 2.5 m$^3$/kg and a concentration of < 0.2 % w/v. In some embodiments, the hyaluronic acid has an intrinsic viscosity of greater than 2.9 m$^3$/kg.

Viscoelasticity is defined as characteristics of a fluid having both viscous and elastic properties. The zero shear viscosity is determined as the steady shear plateau viscosity at vanishing shear rate. For highly viscous formulations, measurement with a controlled stress rheometer is preferred.

The relation between molecular weight and intrinsic viscosity [η] in m$^3$/kg is given through the Mark-Houwink equation:

$$[\eta] = k \cdot (M_{rm})^a$$

with $M_{rm}$ being the molecular mass in MDa
and the coefficients

$$k = 1.3327 \cdot 10^{-4}$$

and
a = 0.6691
which values for k and a having been found as most predictive.

The fluid may be produced by: sterilizing the filling line; adding purified water or water for injection (WFI) to a stainless steel mixing tank; adding salts while mixing; slowly adding HA and mixing until a homogeneous solution/fluid is achieved; adjusting pH value by adding NaOH or HCl, if required, while continuing the mixing process; transferring the solution over a 1 μm pore size filter cartridge to a sterile holding tank; and aseptically filling the solution via sterile filtration into the sterile primary package (monodose or vial). In the case of monodoses, this may be done by a blow-fill-seal (BFS) process.

Preferably, the fluid has at least essentially mucin-free or in other words having a mucin concentration of < 0.3 % w/v. This means that the flow behavior or properties essentially is reached or adjusted by hyaluronan and not by mucin naturally present in the subject's tear fluid and mainly responsible for the flow behavior thereof.

It is preferred that if substances are added that increase the viscosity, they are added towards, or during, or as a final step. The mixing is carried out so as to reach a homogeneous mixture. As an alternative or in addition, it is preferred to initially provide purified water or water for injection as a basis, and then, optionally, electrolytes, buffers and substances which do not increase the viscosity are added at first to the purified water or water for injection.

HA and its production is further described in the monograph of the European Pharmacopoeia 9.0, page 3583 (Sodium Hyaluronate), which is incorporated herein by reference in its entirety.

In one embodiment, the fluid used in the method and kit of the invention has the characteristics listed in Table 1:

**Table 1.**

| Characteristic | Specification | Test Method |
|---|---|---|
| appearance | clear and colorless solution, free from visible impurities | Ph.Eur. |
| pH value | 6.8 - 7.6 | Ph.Eur. |
| osmolality | 240 - 330 mosmol/kg | Ph.Eur. |
| HA concentration | 0.10 - 0.19 % w/v | Ph.Eur. |
| NaCl concentration | 7.6 - 10.5 g/l | Ph.Eur. |
| sterility | Sterile | Ph.Eur. |
| Phosphate concentration | 1.0 - 1.4 mmol/l | Ph.Eur. |

Exemplified Embodiments:

Embodiment 1. A method for reducing or delaying the onset of a condition selected from among an irritation, discomfort, inflammation, immune response, or combination of two or more of the foregoing, at an ocular surface, comprising topically administering a fluid comprising high molecular weight hyaluronic acid to the ocular surface of the eye of a human or non-human animal subject, wherein the hyaluronic acid has an intrinsic viscosity of > 2.5 $m^3$/kg and a concentration of < 0.2 % w/v.

Embodiment 2. The method of embodiment 1, wherein the fluid is administered prophylactically, before the condition exists.

Embodiment 3. The method of embodiment 1, wherein the fluid is administered therapeutically, after the condition exists.

Embodiment 4. The method of any one of embodiments 1 to 3, wherein the condition comprises one or more of the following characteristics: leukocyte invasion at the ocular surface and tears, CD44 upregulation at the ocular surface, and activation of an immune cascade that includes one or more of IL-1, IL-2, IL-5, IL-6, IL-8, CXCL8, IL-10, IL-12, IL-16, IL-33, MCP1, CCL2, MIP1d (also known as CCL15), ENA-78, CXCL5, SILR1, sIL-6R, sgp sEGFR, sTNFR, I-17A, IL-21, IL-22, CXCL9, MIG, CXCL11, I-TAC, CXCL10, IP-10, MIP-1β, CCL4, RANTES, and CCL5.

Embodiment 5. The method of any preceding embodiment, wherein the condition is caused by an external stimulus resulting in a disruption of the smoothness and/or integrity of the ocular surface (*e.g.,* medical therapy, ocular surgery, non-surgical trauma, contact lens wearing, microbial infection, allergen, hapten, toxic agent, or irritative substance).

Embodiment 6. The method of any one of embodiments 1 to 4, wherein the condition is caused by an internal stimulus (*e.g.,* hormonal disturbance, rheumatic disease, epithelial-mesenchymal transition (EMT), or autoimmune disease).

Embodiment 7. The method of any preceding embodiment, wherein the condition is caused by a wound of the eye.

Embodiment 8. The method of embodiment 7, wherein the wound is caused by physical trauma, chemical trauma, or radiation (radiation injury).

Embodiment 9. The method of embodiment 7, wherein the wound is caused by an ocular surgery.

Embodiment 10. The method of embodiment 9, wherein the ocular surgery is selected from among natural or artificial corneal transplantation, corneal implantation (*e.g.,* intracorneal rings (ICRs), and keratoprosthesis), glaucoma surgery, cataract surgery (*e.g.,* phacoemulsification, extrapsular cataract surgery, or intracapsular surgery), refractive surgery (*e.g.,* radial keratotomy or refractive corneal incision), retinal surgery, squint (strabismus) surgery, corrective laser eye surgery (*e.g.,* laser-assisted in situ keratomileusis (LASIK) or photorefractive keratectomy (PRK)), and cross-linking surgery.

Embodiment 11. The method of any preceding embodiment, wherein the inflammation is acute ocular surface inflammation.

Embodiment 12. The method of any one of embodiments 1 to 10, wherein the inflammation is chronic ocular surface inflammation.

Embodiment 13. The method of any one of embodiments 1 to 5, wherein the condition is eye allergy.

Embodiment 14. The method of any one of embodiments 1 to 5, wherein the condition is allergic keratoconjunctivitis (*e.g.,* seasonal or non-seasonal).

Embodiment 15. The method of any preceding embodiment, wherein the discomfort is itchiness or ocular pain.

Embodiment 16. The method of any preceding embodiment, wherein the subject is immunocompromised.

Embodiment 17. The method of any preceding embodiment, wherein the fluid contains no other bioactive agent (*e.g.,* no hydrophobic active ingredient).

Embodiment 18. The method of any preceding embodiment, wherein the fluid contains no other immunodulatory agent, immunosuppressive agent, or antibiotic.

Embodiment 19. The method of any one of embodiments 1 to 14, wherein the fluid further comprises a bioactive agent (*e.g.,* a hydrophobic active ingredient).

Embodiment 20. The method of embodiment 19, wherein the bioactive agent is irritative or damaging to the eye.

Embodiment 21. The method of any one of embodiments 1 to 16, 18, 19, or 20, wherein the fluid further comprises a preservative or detergent.

Embodiment 22. The method of embodiment 21, wherein the preservative or detergent is irritative or damaging to the eye.

Embodiment 23. The method of any preceding embodiment, wherein the fluid is administered before, during, and/or after administration of a composition comprising: a bioactive agent (*e.g.,* a hydrophobic active ingredient), preservative, detergent, or combination of two or more of the foregoing.

Embodiment 24. The method of embodiment 23, wherein the bioactive agent, preservative, detergent, or combination is irritative or damaging to the eye.

Embodiment 25. The method of any one of embodiments 21 to 24, wherein the preservative or detergent is a chemical preservative or oxidative preservative.

Embodiment 26. The method of any one of embodiments 21 to 25, wherein the preservative or detergent is one that kills susceptible microbial cells by disrupting the lipid structure of the microbial cell membrane, thereby increasing microbial cell membrane permeability.

Embodiment 27. The method of any one of embodiments 21 to 26, wherein the preservative or detergent is one that causes damage to the corneal tissues.

Embodiment 28. The method of any one of embodiments 21 to 27, wherein the preservative or detergent is selected from the group consisting of quaternary ammonium preservative (*e.g.,* benzalkonium chloride (BAK)), chlorobutanol, edetate disodium (EDTA), polyquaternarium-1 (*e.g.,* Polyquad™ preservative), stabilized oxidizing agent (*e.g.,* stabilized oxychloro complex (*e.g.,* Purite™ preservative)), ionic-buffered preservative (*e.g.,* sofZia™ preservative), polyhexamethylene biguanide (PHMB), sodium perborate (*e.g.,* GenAqua™ preservative), and sorbate.

Embodiment 29. The method of any preceding embodiment, wherein the fluid is administered directly to the ocular surface as drops or as a wash (*e.g.,* lavage).

Embodiment 30. The method of embodiment 29, wherein 1 to 3 drops are administered, 1 to 3 times per day.

Embodiment 31. The method of any one of embodiments 1 to 29, wherein the inflammation is chronic ocular surface inflammation, and wherein 1 to 3 drops are administered, 4, 5, 6, 7, 8, 9, or 10 or more times per day.

Embodiment 32. The method of any one of embodiments 1 to 28, wherein the fluid is administered indirectly to the ocular surface by a delivery agent (a fluid delivery agent) that is topically administered to the ocular surface or other part of the eye (*e.g.,* a particle that is coated with and/or secretes the fluid on to the ocular surface).

Embodiment 33. The method of any preceding embodiment, wherein the hyaluronic acid has an intrinsic viscosity of > 2.9 m$^3$/kg.

Embodiment 34. The method of any one of embodiments 1 to 16, wherein the fluid further comprises a preservative.

Embodiment 35. The method of any one of embodiments 1 to 16, wherein the fluid does not further comprise a preservative (*i.e.,* the fluid is preservative-free).

Embodiment 36. The method of any one of embodiments 1 to 16, wherein the fluid further comprises an additional glycosaminoglycan (GAG), an electrolyte (*e.g.,* sodium chloride), a buffer (*e.g.,* phosphate buffer), or a combination of two or more of the foregoing.

Embodiment 37. The method of any preceding embodiment, wherein the eye of the subject does not have aqueous tear deficiency (ATD) at the time of said administering (*i.e.,* in the absence of ATD).

Embodiment 38. The method of any preceding embodiment, wherein the eye of the subject does not have qualitative dry eye at the time of said administering (*i.e.,* in the absence of qualitative dry eye).

Embodiment 39. The method of any preceding embodiment, wherein the eye of the subject does not have dry eye syndrome at the time of said administering (*i.e.,* in the absence of aqueous tear deficiency or qualitative dry eye).

Embodiment 40. The method of any preceding embodiment, wherein the subject is not suffering from an aqueous tear deficiency (ATD), and wherein the subject has an ocular surface abnormality (a topographic anomaly) comprising elevations on the cornea or elsewhere on the eye surface for which the normal tear film (tear film of normal surface tension and viscosity) does not cover, resulting in areas of friction at the ocular surface, and wherein the administered fluid reduces the friction.

Embodiment 41. The method of any preceding embodiment, wherein the eye of the subject is in homeostasis at the ocular surface at the time of said administering.

Embodiment 42. The method of any preceding embodiment, further comprising applying a bandage contact lens to the eye before, during, or after said administering.

Embodiment 43. The method of embodiment 42, wherein the fluid is administered and the bandage contact lens is applied after ocular surgery.

Embodiment 44. The method of any preceding embodiment, wherein the administered fluid increases visual performance of the eye.

Embodiment 45. The method of embodiment 44, wherein the fluid is administered before, during, and/or after ocular surgery, and wherein the subject recovers visual performance following the ocular surgery (*i.e.,* recovers greater visual performance following the ocular surgery than the subject would have in the absence of administration of the fluid).

Embodiment 46. The method of any preceding embodiment, wherein the hyaluronic acid has a molecular weight of at least 3 million Daltons.

Embodiment 47. The method of any preceding embodiment, wherein the hyaluronic acid has a molecular weight in the range of 3 million to 4 million Daltons.

Embodiment 48. A kit comprising: the fluid of any one of embodiments 1 to 47, and one or more bandage contact lenses.

The present invention also concerns a fluid (a topical fluid) for topical administration to an ocular surface.

Embodiment 49. A fluid for use in reducing or delaying the onset of a condition selected from among an irritation, discomfort, inflammation, immune response, or combination of two or more of the foregoing, at an ocular surface, wherein the fluid comprises high molecular weight hyaluronic acid having an intrinsic viscosity of $> 2.5$ m$^3$/kg and a concentration of $< 0.2$ % w/v, and wherein the fluid is topically administered to the ocular surface of the eye of a human or non-human animal subject.

Embodiment 50. The fluid of embodiment 49, wherein the fluid is administered prophylactically, before the condition exists.

Embodiment 51. The fluid of embodiment 49, wherein the fluid is administered therapeutically, after the condition exists.

Embodiment 52. The fluid of any one of embodiments 49 to 51, wherein the condition comprises one or more of the following characteristics: leukocyte invasion at the ocular surface and tears, CD44 upregulation at the ocular surface, and activation of an immune cascade that includes one or more of IL-1, IL-2, IL-5, IL-6, IL-8, CXCL8, IL-10, IL-12, IL-16, IL-33, MCP1, CCL2, MIP1d (also known as CCL15), ENA-78, CXCL5, SILR1, sIL-6R, sgp sEGFR, sTNFR, I-17A, IL-21, IL-22, CXCL9, MIG, CXCL11, I-TAC, CXCL10, IP-10, MIP-1$\beta$, CCL4, RANTES, and CCL5.

Embodiment 53. The fluid of any one of embodiments 49 to 51, wherein the condition is caused by an external stimulus resulting in a disruption of the smoothness and/or integrity of the ocular surface (*e.g.,* medical therapy, ocular surgery, non-surgical trauma, contact lens wearing, microbial infection, allergen, hapten, toxic agent, or irritative substance).

Embodiment 54. The fluid of any one of embodiments 49 to 51, wherein the condition is caused by an internal stimulus (*e.g.,* hormonal disturbance, rheumatic disease, epithelial-mesenchymal transition (EMT), or autoimmune disease).

Embodiment 55. The fluid of any one of embodiments 49 to 51, wherein the condition is caused by a wound of the eye.

Embodiment 56. The fluid of embodiment 55, wherein the wound is caused by physical trauma, chemical trauma, or radiation (radiation injury).

Embodiment 57. The fluid of embodiment 55, wherein the wound is caused by an ocular surgery.

Embodiment 58. The fluid of embodiment 57, wherein the ocular surgery is selected from among natural or artificial corneal transplantation, corneal implantation (*e.g.,* intracorneal rings (ICRs), and keratoprosthesis), glaucoma surgery, cataract surgery (e.g., phacoemulsification, extrapsular cataract surgery, or intracapsular surgery), refractive surgery (*e.g.,* radial keratotomy or refractive corneal incision), retinal surgery, squint (strabismus) surgery, corrective laser eye surgery (*e.g.,* laser-assisted in situ keratomileusis (LASIK) or photorefractive keratectomy (PRK)), and cross-linking surgery.

Embodiment 59. The fluid of any one of embodiments 49 to 51, wherein the inflammation is acute ocular surface inflammation.

Embodiment 60. The fluid of any one of embodiments 49 to 51, wherein the inflammation is chronic ocular surface inflammation.

Embodiment 61. The fluid of any one of embodiments 49 to 51, wherein the condition is eye allergy.

Embodiment 62. The fluid of any one of embodiments 49 to51, wherein the condition is allergic keratoconjunctivitis (*e.g.,* seasonal or non-seasonal).

Embodiment 63. The fluid of any one of embodiments 49 to 51, wherein the discomfort is itchiness or ocular pain.

Embodiment 64. The fluid of any one of embodiments 49 to 51, wherein the fluid is administered prophylactically, to prevent or delay the onset of the condition, and wherein the subject is immunocompromised.

Embodiment 65. The fluid of any one of embodiments 49 to 51, wherein the fluid contains no other bioactive agent (*e.g.,* no hydrophobic active ingredient).

Embodiment 66. The fluid of any one of embodiments 49 to 51, wherein the fluid contains no other immunodulatory agent, immunosuppressive agent, or antibiotic.

Embodiments 67. The fluid of any one of embodiments 49 to 51, wherein the fluid further comprises a bioactive agent (*e.g.,* a hydrophobic active ingredient).

Embodiment 68. The fluid of embodiment 67, wherein the bioactive agent is irritative or damaging to the eye.

Embodiment 69. The fluid of any one of embodiments 49 to 51, wherein the fluid further comprises a preservative or detergent.

Embodiment 70. The fluid of embodiment 69, wherein the preservative or detergent is irritative or damaging to the eye.

Embodiment 71. The fluid of any one of embodiments 49 to 51, wherein the fluid is administered before, during, and/or after administration of a composition comprising: a bioactive agent (*e.g.,* a hydrophobic active ingredient), preservative, detergent, or combination of two or more of the foregoing.

Embodiment 72. The fluid of embodiment 71, wherein the bioactive agent, preservative, detergent, or combination is irritative or damaging to the eye.

Embodiment 73. The fluid of embodiment 69, wherein the preservative or detergent is a chemical preservative or oxidative preservative.

Embodiment 74. The fluid of embodiment 69, wherein the preservative or detergent is one that kills susceptible microbial cells by disrupting the lipid structure of the microbial cell membrane, thereby increasing microbial cell membrane permeability.

Embodiment 75. The fluid of embodiment 69, wherein the preservative or detergent is one that causes damage to the corneal tissues.

Embodiment 76. The fluid of embodiment 69, wherein the preservative or detergent is selected from the group consisting of quaternary ammonium preservative (e.g., benzalkonium chloride (BAK)), chlorobutanol, edetate disodium (EDTA), polyquaternarium-1 (*e.g.,* Polyquad™ preservative), stabilized oxidizing agent (*e.g.,* stabilized oxychloro complex (*e.g.,* Purite™ preservative)), ionic-buffered preservative (*e.g.,* sofZia™ preservative), polyhexamethylene biguanide (PHMB), sodium perborate (*e.g.,* GenAqua™ preservative), and sorbate.

Embodiment 77. The fluid of any one of embodiments 49 to 51, wherein the fluid is administered directly to the ocular surface as drops or as a wash.

Embodiment 78. The fluid of embodiment 77, wherein 1 to 3 drops are administered, 1 to 3 times per day.

Embodiment 79. The fluid of any one of embodiments 49 to 51, wherein the inflammation is chronic ocular surface inflammation, and wherein 1 to 3 drops are administered 4, 5, 6, 7, 8, 9, or 10 or more times per day.

Embodiment 80. The fluid of any one of embodiments 49 to 51, wherein the fluid is administered indirectly to the ocular surface by a delivery agent that is topically administered to the ocular surface or other part of the eye (*e.g.,* a particle that is coated with and/or secretes the fluid on to the ocular surface).

Embodiment 81. The fluid of any one of embodiments 49 to 51, wherein the hyaluronic acid has an intrinsic viscosity of > 2.9 $m^3$/kg.

Embodiment 82. The fluid of any one of embodiments 49 to 51, wherein the fluid further comprises a preservative.

Embodiment 83. The fluid of any one of embodiments 49 to 51, wherein the fluid does not further comprise a preservative (*i.e.,* the fluid is preservative-free).

Embodiment 84. The fluid of any one of embodiments 49 to 51, wherein the fluid further comprises an additional glycosaminoglycan (GAG), an electrolyte (*e.g.,* sodium chloride), a buffer (*e.g.,* phosphate buffer), or a combination of two or more of the foregoing.

Embodiment 85. The fluid of any one of embodiments 49 to 51, wherein the eye of the subject does not have aqueous tear deficiency (ATD) at the time of said administering (*i.e.,* in the absence of ATD).

Embodiment 86. The fluid of any one of embodiments 49 to 51, wherein the eye of the subject does not have qualitative dry eye at the time of said administering (*i.e.,* in the absence of qualitative dry eye).

Embodiment 87. The fluid of any one of embodiments 49 to 51, wherein the eye of the subject does not have dry eye syndrome at the time of said administering (*i.e.,* in the absence of aqueous tear deficiency or qualitative dry eye).

Embodiment 88. The fluid of any one of embodiments 49 to 51, wherein the subject is not suffering from an aqueous tear deficiency (ATD), and wherein the subject has an ocular surface abnormality (a topographic anomaly) comprising elevations on the cornea or elsewhere on the eye surface for which the normal tear film (tear film of normal surface tension and viscosity) does not cover, resulting in areas of friction at the ocular surface, and wherein the administered fluid reduces the friction.

Embodiment 89. The fluid of any of embodiments 49 to 51, wherein the eye of the subject is in homeostasis at the ocular surface at the time of said administering.

Embodiment 90. The fluid of any one of embodiments 49 to 51, further comprising applying a bandage contact lens to the eye before, during, or after said administering.

Embodiment 91. The fluid of embodiment 90, wherein the fluid is administered and the bandage contact lens is applied after ocular surgery.

Embodiment 92. The fluid of any one of embodiments 49 to 51, wherein the administered fluid increases visual performance of the eye.

Embodiment 93. The fluid of embodiment 92, wherein the fluid is administered before, during, and/or after ocular surgery, and wherein the subject recovers visual performance following the ocular surgery.

Embodiment 94. The fluid of any any one of embodiments 49 to 51, wherein the hyaluronic acid has a molecular weight of at least 3 million Daltons.

Embodiment 95. The fluid of any any one of embodiments 49 to 51, wherein the hyaluronic acid has a molecular weight in the range of 3 million to 4 million Daltons.

Definitions

The term "a," "an," "the" and similar terms used in the context of the present invention (especially in the context of the claims) are to be construed to cover both the singular and plural unless otherwise indicated herein or clearly contradicted by the context. Thus, for example, reference "a cell" or "a compound" should be construed to cover both a singular cell or singular compound and a plurality of cells and a plurality of compounds unless indicated otherwise or clearly contradicted by the context. Similarly, the word "or" is intended to include "and" unless the context clearly indicates otherwise. The abbreviation, "e.g." is derived from the Latin *exempli gratia,* and is used herein to indicate a non-limiting example. Thus, the abbreviation "*e.g.*" is synonymous with the term "for example."

The term "isolated," when used as a modifier of a composition, means that the compositions are made by human intervention or are separated from their naturally occurring *in vivo* environment. Generally, compositions so separated are substantially free of one or more materials with which they normally associate with in nature, for example, one or more protein, nucleic acid, lipid, carbohydrate, cell membrane. A "substantially pure" molecule can be combined with one or more other molecules. Thus, the term "substantially pure" does not exclude combinations of compositions. Substantial purity can be at least about 60% or more of the molecule by mass. Purity can also be about 70% or 80% or more, and can be greater, for example, 90% or more. Purity can be determined by any appropriate method, including, for example, UV spectroscopy, chromatography (*e.g.,* HPLC, gas phase), gel electrophoresis (*e.g.,* silver or coomassie staining) and sequence analysis (for nucleic acid and peptide).

As used herein, the term "homeostasis" refers to the capacity of a physiological system to maintain internal stability, or to the state of stability itself, owing to the coordinated response of its parts to any situation or stimulus that would tend to disturb its normal, non-pathological condition or function.

As used herein, the term "hyaluronic acid" (HA) refers to the glycosaminoglycan composed of disaccharide repeats of N-acetylglucosamine and glucuronic acid found in nature, also known as hyaluronan (*e.g.,* the straight chain, glycosaminoglycan polymer composed of repeating units of the disaccharide [-D-glucuronic acid-b1,3-N-acetyl-D-glucosamine-b1,4-]*n*), as well as derivatives of hyaluronan having chemical modifications such as esters of hyaluronan, amide derivatives, alkyl-amine derivatives, low molecular weight and high molecular weight forms of hyaluronans, and cross-linked forms such as hylans. Thus, the disaccharide chain may be linear or non-linear. Hyaluronan can be cross-linked by attaching cross-linkers such as thiols, methacrylates, hexadecylamides, and tyramines. Hyaluronan can also be cross-linked directly with formaldehyde and divinylsulfone. Examples of hylans include, but are not limited to, hylan A, hylan A (a formaldehyde cross-linked glycosaminoglycan polymer), hylan B (a divinylsulfone cross-linked glycosaminoglycan polymer), and hylan G-F 20 (Cowman MK et al., Carbohydrate Polymers 2000, 41:229-235; Takigami S et al., Carbohydrate Polymers, 1993, 22:153-160; Balazs EA et al., "Hyaluronan, its cross-linked derivative-Hylan-and their medical applications", in Cellulosics Utilization: Research and Rewards in Cellulosics, Proceedings of Nisshinbo International Conference on Cellulosics Utilization in the Near Future (Eds Inagaki, H and Phillips GO), Elsevier Applied Science (1989), NY, pp.233-241; Koehler L et al., Scientific Reports, 2017, 7, article no. 1210; and Pavan M et al.,

Carbohydr Polym, 2013, 97(2): 321-326; which are each incorporated herein by reference in their entirety).

The term "hyaluronic acid" or HA includes HA itself and pharmaceutically acceptable salts thereof. The HA can be formulated into pharmaceutically-acceptable salt forms. Pharmaceutically-acceptable salts of HA can be prepared using conventional techniques.

The term "high molecular weight" or "HMW" in the context of hyaluronic acid of the invention refers to hyaluronic acid having an intrinsic viscosity of > 2.5 m$^3$/kg as determined by the method of the European Pharmacopoeia 9.0, "Sodium Hyaluronate", page 3584 (which is incorporated herein by reference in its entirety). Briefly, the intrinsic viscosity [$\eta$] is calculated by linear least-squares regression analysis using the Martin equation: $\log_{10} (n_r\text{-}1/c) = \log_{10} [\eta] + \kappa[\eta]$ $c$. In some embodiments, the high molecular weight hyaluronic acid has an intrinsic viscosity of greater than 2.9 m$^3$/kg.

As used herein, the term "immunocompromised" refers to a subject with an innate, acquired, or induced inability to develop a normal immune response. An immunocompromised subject, therefore, has a weakened or impaired immune system relative to one of a normal subject. A subject with a weakened or impaired immune system has an "immunodeficiency" or "immunocompromised condition," which is associated with a primary or secondary deficiency, induced or non-induced, in one or more of the elements of the normal immune defense system. An immunocompromised condition may be due to a medical treatment, *e.g.,* radiation therapy, chemotherapy or other immunosuppressing treatment, such as induced by treatment with steroids, cyclophosphamide, azathioprine, methotrexate, cyclosporine or rapamycin, in particular in relation to cancer treatment or the treatment or prevention of transplant rejection. The presence of an immunocompromised condition in a subject can be diagnosed by any suitable technique known to persons of skill the art. Strong indicators that an immunocompromised condition may be present is when rare diseases occur or the subject gets ill from organisms that do not normally cause diseases, especially if the subject gets repeatedly infected. Other possibilities are typically considered, such as recently acquired infections, for example, HIV, hepatitis, tuberculosis, *etc.* Generally, however, definitive diagnoses are based on laboratory tests that determine the exact nature of the immunocompromised condition. Most tests are performed on blood samples. Blood contains antibodies, lymphocytes, phagocytes, and complement components, all of the major immune components that might cause immunodeficiency. A blood cell count can be used to determine if the number of phagocytic cells or lymphocytes is below normal. Lower than normal counts of either of these two cell types correlates with an immunocompromised condition. The blood cells are also typically checked for their appearance. Sometimes, a subject may have normal cell counts, but the cells are structurally defective. If the lymphocyte cell count is low, further testing is usually conducted to determine whether any particular type of lymphocyte is lower than normal. A lymphocyte proliferation test may be conducted to determine if the lymphocytes can respond to stimuli. The failure to respond to stimulants correlates with an immunocompromised condition. Antibody levels and complement levels can also be determined for diagnosing the presence of an immunocompromised condition.

As used herein, the term "ocular surface" refers to the structures of the eye and adnexa, including the cornea, conjunctiva, eyelids, eyelashes, tear film, main and accessory lacrimal glands, and the meibomian glands. Thus, the tears, both in terms of the individual components at the site of production, and as a film on the ocular surface, are included with the term "ocular surface" (see Craig JP et al., "TFOS DEWS II Definition and Classification Report", The Ocular Surface, 2017, 15:276-283, which is incorporated herein by reference in its entirety). The fluid may be topically administered to one or more parts of the ocular surface, including, for example, the entire ocular surface.

"Pharmaceutically acceptable salt" includes both acid and base addition salts. A pharmaceutically acceptable salt of HA or any one of the other compounds described herein is intended to encompass any and all pharmaceutically suitable salt forms. Preferred pharmaceutically acceptable salts described herein are pharmaceutically acceptable acid addition salts and pharmaceutically acceptable base addition salts.

"Pharmaceutically acceptable acid addition salt" refers to those salts which retain the biological effectiveness and properties of the free bases, which are not biologically or otherwise undesirable, and which are formed with inorganic acids such as hydrochloric acid, hydrobromic acid, sulfuric acid, nitric acid, phosphoric acid, hydroiodic acid, hydrofluoric acid, phosphorous acid, and the like. Also included are salts that are formed with organic acids such as aliphatic mono- and dicarboxylic acids, phenyl-substituted alkanoic acids, hydroxy alkanoic acids, alkanedioic acids, aromatic acids, aliphatic and. aromatic sulfonic acids, etc. and include, for example, acetic acid, trifluoroacetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like. Exemplary salts thus include sulfates, pyrosulfates, bisulfates, sulfites, bisulfites, nitrates, phosphates, monohydrogenphosphates, dihydrogenphosphates, metaphosphates, pyrophosphates, chlorides, bromides, iodides, acetates, trifluoroacetates, propionates, caprylates, isobutyrates, oxalates, malonates, succinate suberates, sebacates, fumarates, maleates, mandelates, benzoates, chlorobenzoates, methylbenzoates, dinitrobenzoates, phthalates, benzenesulfonates, toluenesulfonates, phenylacetates, citrates, lactates, malates, tartrates, methanesulfonates, and the like. Also contemplated are salts of amino acids, such as arginates, gluconates, and galacturonates (see, for example, Berge S. M. et al., "Pharmaceutical Salts," Journal of Pharmaceutical Science, 66:1-19 (1997), which is hereby incorporated by reference in its entirety). Acid addition salts of basic compounds may be prepared by contacting the free base forms with a sufficient amount of the desired acid to produce the salt according to methods and techniques with which a skilled artisan is familiar.

"Pharmaceutically acceptable base addition salt" refers to those salts that retain the biological effectiveness and properties of the free acids, which are not biologically or otherwise undesirable. These salts are prepared from addition of an inorganic base or an organic base to the free acid. Pharmaceutically acceptable base addition salts may be formed with metals or amines, such as alkali and alkaline earth metals or organic amines. Salts derived from inorganic bases include, but are not limited to, sodium, potassium, lithium, ammonium, calcium, magnesium, iron, zinc, copper, manganese, aluminum salts and the like. Salts derived from organic bases include, but are not limited to, salts of primary, secondary, and tertiary amines, substituted amines including naturally occurring substituted amines, cyclic amines and basic ion exchange resins, for example, isopropylamine, trimethylamine, diethylamine, triethylamine, tripropylamine, ethanolamine, diethanolamine, 2-dimethylaminoethanol, 2-diethylaminoethanol, dicyclohexylamine, lysine, arginine, histidine, caffeine, procaine, N,N-dibenzylethylenediamine, chloroprocaine, hydrabamine, choline, betaine, ethylenediamine, ethylenedianiline, N-methylglucamine, glucosamine, methylglucamine, theobromine, purines, piperazine, piperidine, N-ethylpiperidine, polyamine resins and the like. See Berge *et al., supra.* In some embodiments, the pharmaceutically acceptable salt is sodium salt (see "Sodium Hyaluronate" at page 3583 of European Pharmacopoeia 9.0, which is incorporated herein by reference).

As used herein, the terms "subject", "patient", and "individual" refer to a human or non-human animal. A subject also refers to, for example, primates (*e.g.,* humans), cows, sheep, goats, horses, dogs, cats, rabbits, rats, mice, fish, birds and the like. In some embodiments, the subject is a mammal. In some embodiments, the subject is a human. In some embodiments, the subject is a bird or fish. Thus, the methods may be carried out in the medical setting and the veterinary setting. The non-human animal subject may be, for example, a pet or an animal model of an ocular or non-ocular disease.

In some embodiments, the eye of the subject does not have aqueous tear deficiency (ATD) at the time of said administering (*i.e.,* in the absence of ATD).

In some embodiments, the eye of the subject does not have qualitative dry eye at the time of said administering (*i.e.,* in the absence of qualitative dry eye).

In some embodiments, the eye of the subject does not have dry eye syndrome at the time of said administering (*i.e.,* in the absence of aqueous tear deficiency or qualitative dry eye).

In some embodiments, the subject is not suffering from an aqueous tear deficiency (ATD), and wherein the subject has an ocular surface abnormality (a topographic anomaly) comprising elevations on the cornea or elsewhere on the eye surface for which the normal tear film (tear film of normal surface tension and viscosity) does not cover, resulting in areas of friction at the ocular surface, and wherein the administered fluid reduces the friction.

In some embodiments, the subject is immunocompromised, *i.e.,* is in an immunocompromised condition.

The phrase "topical administration" is used herein in its conventional sense to mean topical delivery to the desired anatomical site, such as the ocular surface. The fluid comprising high molecular weight hyaluronic acid may be applied directly or indirectly to the ocular surface by any manner that allows an effective amount of the fluid and ocular surface to make contact. For example, the fluid may be applied directly to the ocular surface, such as via eye drops or lavage, or applied indirectly via a delivery agent (*i.e.,* a fluid delivery agent) that is brought into contact with the ocular surface or other part of the eye. An example of a delivery agent is a particle (*e.g.,* microparticles or nanoparticles) that is coated with the fluid and/or releases the fluid onto the ocular surface. Such particles may be composed of various materials, such as natural or synthetic polymers. In some embodiments, the delivery agent may itself be administered as drops.

The invention is described only exemplarily by the embodiments in the description and drawings and is not limited thereto but rather includes all variations, modifications, substitutions, and combinations the expert may take from the complete documents of this application under consideration of and/or combination with his specific knowledge.

All patents, patent applications, provisional applications, and publications referred to or cited herein are incorporated by reference in their entirety, including all figures and tables, to the extent they are not inconsistent with the explicit teachings of this specification.

Following is an example that illustrates procedures for practicing the invention. These examples should not be construed as limiting. All percentages are by weight and all solvent mixture proportions are by volume unless otherwise noted.

Example 1-A Multi-Center, Multi-National Prospective Clinical Study on Patients with Severe Dry Eyes - The HYLAN M Study

A multi-center, multi-national prospective, randomized clinical study on patients with severe dry eyes (according to the ODISSEY primary criteria) is being conducted in 12 study centers in 9 countries. Within the HYLAN M study, the patients are randomized in two groups, one staying with the most effective individual patient treatment identified before, the other one switched to high molecular weight hyaluronic acid eye drops (Comfort Shield® preservative-free sodium hyaluronate eye drops (i.com medical GmbH, Munich, Germany)), which corresponds to the embodiment of Table 1 herein.

These patients (192 enrolled) have already received the best treatment their ophthalmologists could offer. All of the patients had been under "stable" therapy at the time of their inclusion into the study, *i.e.,* their therapy has not been changed over a defined period of time prior to inclusion into this study. The patients are randomized into two groups, one group of patients remaining with their current therapy for dry eye syndrome, and the second group of patients treated with drops of the fluid described above (Comfort Shield® eye drops) in place of their tear substitute.

Study objectives include: (1) comparison of objective and subjective symptoms of dry eye under treatment with Comfort Shield® eye drops versus the tear substitute eye dropw which the patients has been treated with before presenting to the investigator (=current therapy) in severe dry eye conditions; and (2) to observe objective performance, patients' subjective acceptance and adverse events of the eye drops. For each patient, both eyes are examined, and the eye with higher corneal fluorescein staining score at baseline examination is evaluated.

In one of the study centers, all commercially available tear substitutes did not result in adequate relieve of signs and symptoms, by autologous serum eye drops. They have included 11 patients with autologous serum eye drop treatment into the study. Out of these 11 patients, 6 have been randomized to the Comfort Shield® group, *i.e.* the use of autologous serum eye drops was replaced by Comfort Shield® over a period of 8 weeks. Out of these 6 patients, 2 discontinued their participation in the study, because Comfort Shield® eye drops did not provide adequate relief of symptoms. Two continued with the Comfort Shield® eye drop therapy over the eight weeks of the study, but preferred to return to their original therapy with autologous serum. The remaining two patients preferred Comfort Shield® eye drops over the autologous serum eye drops and decided to use the Comfort Shield® eye drops beyond the study.

It should be understood that the examples and embodiments described herein are for illustrative purposes only and that various modifications or changes in light thereof will be suggested to persons skilled in the art and are to be included within the spirit and purview of this application and the scope of the appended claims. In addition, any elements or limitations of any invention or embodiment thereof disclosed herein can be combined with any and/or all other elements or limitations (individually or in any combination) or any other invention or embodiment thereof disclosed herein, and all such combinations are contemplated with the scope of the invention without limitation thereto.

EMBODIMENTS

1. A fluid for use in reducing or delaying the onset of a condition selected from among an irritation, discomfort, inflammation, immune response, or combination of two or more of the foregoing, at an ocular surface, wherein the fluid comprises high molecular weight hyaluronic acid having an intrinsic viscosity of > 2.5 m$^3$/kg and a concentration of < 0.2 % w/v, and wherein the fluid is topically administered to the ocular surface of the eye of a human or non-human animal subject.

2. The fluid of embodiment 1, wherein the fluid is administered prophylactically, before the condition exists.

3. The fluid of embodiment 1, wherein the fluid is administered therapeutically, after the condition exists.

4. The fluid of any one of embodiments 1 to 3, wherein the condition comprises one or more of the following characteristics: leukocyte invasion at the ocular surface and tears, CD44 upregulation at the ocular surface, and activation of an immune cascade that includes one or more of IL-1, IL-2, IL-5, IL-6, IL-8, CXCL8, IL-10, IL-12, IL-16, IL-33, MCP1, CCL2, MIP1d (also known as CCL15), ENA-78, CXCL5, SILR1, sIL-6R, sgp sEGFR, sTNFR, I-17A, IL-21, IL-22, CXCL9, MIG, CXCL11, I-TAC, CXCL10, IP-10, MIP-1β, CCL4, RANTES, and CCL5.

5. The fluid of any one of embodiments 1 to 3, wherein the condition is caused by an external stimulus resulting in a disruption of the smoothness and/or integrity of the ocular surface (e.g., medical therapy, ocular surgery, non-surgical trauma, contact lens wearing, microbial infection, allergen, hapten, toxic agent, or irritative substance).

6. The fluid of any one of embodiments 1 to 3, wherein the condition is caused by an internal stimulus (*e.g.,* hormonal disturbance, rheumatic disease, epithelial-mesenchymal transition (EMT), or autoimmune disease).

7. The fluid of any one of embodiments 1 to 3, wherein the condition is caused by a wound of the eye.

8. The fluid of embodiment 7, wherein the wound is caused by physical trauma, chemical trauma, or radiation (radiation injury).

9. The fluid of embodiment 7, wherein the wound is caused by an ocular surgery.

10. The fluid of embodiment 9, wherein the ocular surgery is selected from among natural or artificial corneal transplantation, corneal implantation (*e.g.,* intracorneal rings (ICRs), and keratoprosthesis), glaucoma surgery, cataract surgery (*e.g.,* phacoemulsification, extrapsular cataract surgery, or intracapsular surgery), refractive surgery (*e.g.,* radial keratotomy or refractive corneal incision), retinal surgery, squint (strabismus) surgery, corrective laser eye surgery (*e.g.,* laser-assisted in situ keratomileusis (LASIK) or photorefractive keratectomy (PRK)), and cross-linking surgery.

11. The fluid of any one of embodiments 1 to 3, wherein the inflammation is acute ocular surface inflammation.

12. The fluid of any one of embodiments 1 to 3, wherein the inflammation is chronic ocular surface inflammation.

13. The fluid of any one of embodiments 1 to 3, wherein the condition is eye allergy.

14. The fluid of any one of embodiments 1 to 3, wherein the condition is allergic keratoconjunctivitis (*e.g.,* seasonal or non-seasonal).

15. The fluid of any one of embodiments 1 to 3, wherein the discomfort is itchiness or ocular pain.

16. The fluid of any one of embodiments 1 to 3, wherein the fluid is administered prophylactically, to prevent or delay the onset of the condition, and wherein the subject is immunocompromised.

17. The fluid of any one of embodiments 1 to 3, wherein the fluid contains no other bioactive agent (*e.g.,* no hydrophobic active ingredient).

18. The fluid of any one of embodiments 1 to 3, wherein the fluid contains no other immunodulatory agent, immunosuppressive agent, or antibiotic.

19. The fluid of any one of embodiments 1 to 3, wherein the fluid further comprises a bioactive agent (*e.g.,* a hydrophobic active ingredient).

20. The fluid of embodiment 19, wherein the bioactive agent is irritative or damaging to the eye.

21. The fluid of any one of embodiments 1 to 3, wherein the fluid further comprises a preservative or detergent.

22. The fluid of embodiment 21, wherein the preservative or detergent is irritative or damaging to the eye.

23. The fluid of any one of embodiments 1 to 3, wherein the fluid is administered before, during, and/or after administration of a composition comprising: a bioactive agent (*e.g.,* a hydrophobic active ingredient), preservative, detergent, or combination of two or more of the foregoing.

24. The fluid of embodiment 23, wherein the bioactive agent, preservative, detergent, or combination is irritative or damaging to the eye.

25. The fluid of embodiment 21, wherein the preservative or detergent is a chemical preservative or oxidative preservative.

26. The fluid of embodiment 21, wherein the preservative or detergent is one that kills susceptible microbial cells by disrupting the lipid structure of the microbial cell membrane, thereby increasing microbial cell membrane permeability.

27. The fluid of embodiment 21, wherein the preservative or detergent is one that causes damage to the corneal tissues.

28. The fluid of embodiment 21, wherein the preservative or detergent is selected from the group consisting of quaternary ammonium preservative (e.g., benzalkonium chloride (BAK)), chlorobutanol, edetate disodium (EDTA), polyquaternarium-1 (*e.g.,* Polyquad™ preservative), stabilized oxidizing agent (*e.g.,* stabilized oxychloro complex (*e.g.,* Purite™ preservative)), ionic-buffered preservative (*e.g.,* sofZia™ preservative), polyhexamethylene biguanide (PHMB), sodium perborate (*e.g.,* GenAqua™ preservative), and sorbate.

29. The fluid of any one of embodiments 1 to 3, wherein the fluid is administered directly to the ocular surface as drops or as a wash.

30. The fluid of embodiment 29, wherein 1 to 3 drops are administered, 1 to 3 times per day.

31. The fluid of any one of embodiments 1 to 3, wherein the inflammation is chronic ocular surface inflammation, and wherein 1 to 3 drops are administered 4, 5, 6, 7, 8, 9, or 10 or more times per day.

32. The fluid of any one of embodiments 1 to 3, wherein the fluid is administered indirectly to the ocular surface by a delivery agent that is topically administered to the ocular surface or other part of the eye (*e.g.,* a particle that is coated with and/or secretes the fluid on to the ocular surface).

33. The fluid of any one of embodiments 1 to 3, wherein the hyaluronic acid has an intrinsic viscosity of > 2.9 $m^3$/kg.

34. The fluid of any one of embodiments 1 to 3, wherein the fluid further comprises a preservative.

35. The fluid of any one of embodiments 1 to 3, wherein the fluid does not further comprise a preservative (*i.e.,* the fluid is preservative-free).

36. The fluid of any one of embodiments 1 to 3, wherein the fluid further comprises an additional glycosaminoglycan (GAG), an electrolyte (*e.g.,* sodium chloride), a buffer (*e.g.,* phosphate buffer), or a combination of two or more of the foregoing.

37. The fluid of any one of embodiments 1 to 3, wherein the eye of the subject does not have aqueous tear deficiency (ATD) at the time of said administering (*i.e.,* in the absence of ATD).

38. The fluid of any one of embodiments 1 ot 3, wherein the eye of the subject does not have qualitative dry eye at the time of said administering (*i.e.,* in the absence of qualitative dry eye).

39. The fluid of any one of embodiments 1 to 3, wherein the eye of the subject does not have dry eye syndrome at the time of said administering (*i.e.,* in the absence of aqueous tear deficiency or qualitative dry eye).

40. The fluid of any one of embodiments 1 to 3, wherein the subject is not suffering from an aqueous tear deficiency (ATD), and wherein the subject has an ocular surface abnormality (a topographic anomaly) comprising elevations on the cornea or elsewhere on the eye surface for which the normal tear film (tear film of normal surface tension and viscosity) does not cover, resulting in areas of friction at the ocular surface, and wherein the administered fluid reduces the friction.

41. The fluid of any of embodiments 1 to 3, wherein the eye of the subject is in homeostasis at the ocular surface at the time of said administering.

42. The fluid of any one of embodiments 1 to 3, further comprising applying a bandage contact lens to the eye before, during, or after said administering.

43. The fluid of embodiment 42, wherein the fluid is administered and the bandage contact lens is applied after ocular surgery.

44. The fluid of any one of embodiments 1 to 3, wherein the administered fluid increases visual performance of the eye.

45. The fluid of embodiment 44, wherein the fluid is administered before, during, and/or after ocular surgery, and wherein the subject recovers visual performance following the ocular surgery.

46. The fluid of any any one of embodiments 1 to 3, wherein the hyaluronic acid has a molecular weight of at least 3 million Daltons.

47. The fluid of any any one of embodiments 1 to 3, wherein the hyaluronic acid has a molecular weight in the range of 3 million to 4 million Daltons.

48. A kit comprising: the fluid of any one of embodiments 1 to 3, and one or more bandage contact lenses.

49. A method for reducing or delaying the onset of a condition selected from among an irritation, discomfort, inflammation, immune response, or combination of two or more of the foregoing, at an ocular surface, comprising topically administering a fluid comprising high molecular weight hyaluronic acid to the ocular surface of the eye of a human or non-human animal subject, wherein the hyaluronic acid has an intrinsic viscosity of > 2.5 m$^3$/kg and a concentration of < 0.2 % w/v.

50. The method of embodiment 49, wherein the fluid is administered prophylactically, before the condition exists.

51. The method of embodiment 49, wherein the fluid is administered therapeutically, after the condition exists.

52. The method of any one of embodiments 49 to 51, wherein the condition comprises one or more of the following characteristics: leukocyte invasion at the ocular surface and tears, CD44 upregulation at the ocular surface, and activation of an immune cascade that includes one or more of IL-1, IL-2, IL-5, IL-6, IL-8, CXCL8, IL-10, IL-12, IL-16, IL-33, MCP1, CCL2, MIP1d (also known as CCL15), ENA-78, CXCL5, SILR1, sIL-6R, sgp sEGFR, sTNFR, I-17A, IL-21, IL-22, CXCL9, MIG, CXCL11, I-TAC, CXCL10, IP-10, MIP-1β, CCL4, RANTES, and CCL5.

53. The method of any one of embodiments 49 to 51, wherein the condition is caused by an external stimulus resulting in a disruption of the smoothness and/or integrity of the ocular surface (*e.g.,* medical therapy, ocular surgery, non-surgical trauma, contact lens wearing, microbial infection, allergen, hapten, toxic agent, or irritative substance).

54. The method of any one of embodiments 49 to 51, wherein the condition is caused by an internal stimulus (*e.g.,* hormonal disturbance, rheumatic disease, epithelial-mesenchymal transition (EMT), or autoimmune disease).

55. The method of any one of embodiments 49 to 51, wherein the condition is caused by a wound of the eye.

56. The method of embodiment 55, wherein the wound is caused by physical trauma, chemical trauma, or radiation (radiation injury).

57. The method of embodiment 55, wherein the wound is caused by an ocular surgery.

58. The method of embodiment 57, wherein the ocular surgery is selected from among natural or artificial corneal transplantation, corneal implantation (*e.g.,* intracorneal rings (ICRs), and keratoprosthesis), glaucoma surgery, cataract surgery (*e.g.,* phacoemulsification, extrapsular cataract surgery, or intracapsular surgery), refractive surgery (*e.g.,* radial keratotomy or refractive corneal incision), retinal surgery, squint (strabismus) surgery, corrective laser eye surgery (*e.g.,* laser-assisted in situ keratomileusis (LASIK) or photorefractive keratectomy (PRK)), and cross-linking surgery.

59. The method of any one of embodiments 49 to 51, wherein the inflammation is acute ocular surface inflammation.

60. The method of any one of embodiments 49 to 51, wherein the inflammation is chronic ocular surface inflammation.

61. The method of any one of embodiments 49 to 51, wherein the condition is eye allergy.

62. The method of any one of embodiments 49 to 51, wherein the condition is allergic keratoconjunctivitis (*e.g.,* seasonal or non-seasonal).

63. The method of any one of embodiments 49 to 51, wherein the discomfort is itchiness or ocular pain.

64. The method of any one of embodiments 49 to 51, wherein the fluid is administered prophylactically, to prevent or delay the onset of the condition, and wherein the subject is immunocompromised.

65. The method of any one of embodiments 49 to 51, wherein the fluid contains no other bioactive agent (*e.g.,* no hydrophobic active ingredient).

66. The method of any one of embodiments 49 to 51, wherein the fluid contains no other immunodulatory agent, immunosuppressive agent, or antibiotic.

67. The method of any one of embodiments 49 to 51, wherein the fluid further comprises a bioactive agent (*e.g.,* a hydrophobic active ingredient).

68. The method of embodiment 67, wherein the bioactive agent is irritative or damaging to the eye.

69. The method of any one of embodiments 49 to 51, wherein the fluid further comprises a preservative or detergent.

70. The method of embodiment 69, wherein the preservative or detergent is irritative or damaging to the eye.

71. The method of any one of embodiments 49 to 51, wherein the fluid is administered before, during, and/or after administration of a composition comprising: a bioactive agent (*e.g.,* a hydrophobic active ingredient), preservative, detergent, or combination of two or more of the foregoing.

72. The method of embodiment 71, wherein the bioactive agent, preservative, detergent, or combination is irritative or damaging to the eye.

73. The method of embodiment 69, wherein the preservative or detergent is a chemical preservative or oxidative preservative.

74. The method of embodiment 69, wherein the preservative or detergent is one that kills susceptible microbial cells by disrupting the lipid structure of the microbial cell membrane, thereby increasing microbial cell membrane permeability.

75. The method of embodiment 69, wherein the preservative or detergent is one that causes damage to the corneal tissues.

76. The method of embodiment 69, wherein the preservative or detergent is selected from the group consisting of quaternary ammonium preservative (*e.g.,* benzalkonium chloride (BAK)), chlorobutanol, edetate disodium (EDTA), polyquaternarium-1 (*e.g.,* Polyquad™ preservative), stabilized oxidizing agent (*e.g.,* stabilized oxychloro complex (*e.g.,* Purite™ preservative)), ionic-buffered preservative (*e.g.,* sofZia™ preservative), polyhexamethylene biguanide (PHMB), sodium perborate (*e.g.,* GenAqua™ preservative), and sorbate.

77. The method of any one of embodiments 49 to 51, wherein the fluid is administered directly to the ocular surface as drops or as a wash.

78. The method of embodiment 77, wherein 1 to 3 drops are administered, 1 to 3 times per day.

79. The method of any one of embodiments 49 to 51, wherein the inflammation is chronic ocular surface inflammation, and wherein 1 to 3 drops are administered 4, 5, 6, 7, 8, 9, or 10 or more times per day.

80. The method of any one of embodiments 49 to 51, wherein the fluid is administered indirectly to the ocular surface by a delivery agent that is topically administered to the ocular surface or other part of the eye (*e.g.,* a particle that is coated with and/or secretes the fluid on to the ocular surface).

81. The method of any one of embodiments 49 to 51, wherein the hyaluronic acid has an intrinsic viscosity of > 2.9 $m^3$/kg.

82. The method of any one of embodiments 49 to 51, wherein the fluid further comprises a preservative.

83. The method of any one of embodiments 49 to 51, wherein the fluid does not further comprise a preservative (*i.e.,* the fluid is preservative-free).

84. The method of any one of embodiments 49 to 51, wherein the fluid further comprises an additional glycosaminoglycan (GAG), an electrolyte (*e.g.,* sodium chloride), a buffer (*e.g.,* phosphate buffer), or a combination of two or more of the foregoing.

85. The method of any one of embodiments 49 to 51, wherein the eye of the subject does not have aqueous tear deficiency (ATD) at the time of said administering (*i.e.,* in the absence of ATD).

86. The method of any one of embodiments 49 to 51, wherein the eye of the subject does not have qualitative dry eye at the time of said administering (*i.e.,* in the absence of qualitative dry eye).

87. The method of any one of embodiments 49 to 51, wherein the eye of the subject does not have dry eye syndrome at the time of said administering (*i.e.,* in the absence of aqueous tear deficiency or qualitative dry eye).

88. The method of any one of embodiments 49 to 51, wherein the subject is not suffering from an aqueous tear deficiency (ATD), and wherein the subject has an ocular surface abnormality (a topographic anomaly) comprising elevations on the cornea or elsewhere on the eye surface for which the normal tear film (tear film of normal surface tension and viscosity) does not cover, resulting in areas of friction at the ocular surface, and wherein the administered fluid reduces the friction.

89. The method of any of embodiments 49 to 51, wherein the eye of the subject is in homeostasis at the ocular surface at the time of said administering.

90. The method of any one of embodiments 49 to 51, further comprising applying a bandage contact lens to the eye before, during, or after said administering.

91. The method of embodiment 90, wherein the fluid is administered and the bandage contact lens is applied after ocular surgery.

92. The method of any one of embodiments 49 to 51, wherein the administered fluid increases visual performance of the eye.

93. The method of embodiment 92, wherein the fluid is administered before, during, and/or after ocular surgery, and wherein the subject recovers visual performance following the ocular surgery.

94. The method of any one of embodiments 49 to 51, wherein the hyaluronic acid has a molecular weight of at least 3 million Daltons.

95. The method of any one of embodiments 49 to 51, wherein the hyaluronic acid has a molecular weight in the range of 3 million to 4 million Daltons.


**Claims**

1. A fluid for use in treating ocular pain, wherein the fluid comprises high molecular weight hyaluronic acid having an intrinsic viscosity of > 2.5 $m^3$/kg and a concentration of < 0.2 % w/v, wherein the fluid is for topical administration to the ocular surface of the eye of a human or non-human animal subject, and wherein the cause of the pain is unknown.

2. The fluid for use according to claim 1, wherein the hyaluronic acid has an intrinsic viscosity of > 2.9 $m^3$/kg.

3. The fluid for use according to claim 1 or 2, wherein the hyaluronic acid has a molecular weight of at least 3 million Daltons.

4. The fluid for use according to any one of claims 1 to 3, wherein the hyaluronic acid has a molecular weight in the range of 3 million to 4 million Daltons.

5. The fluid for use according to any of claims 1 to 4, wherein the fluid contains no other bioactive agent.

6. The fluid for use according to claim 5, wherein the fluid contains no hydrophobic active ingredient.

7. The fluid for use according to any one of claims 1 to 4, wherein the fluid contains no other immunodulatory agent, immunosuppressive agent, or antibiotic.

8. The fluid for use according to any one of claims 1 to 7, wherein the fluid is for administration directly to the ocular surface as drops or as a wash.

9. The fluid for use according to claim 8, wherein 1 to 3 drops are administered, 1 to 3 times per day.

10. The fluid for use according to any one of claims 1 to 9, wherein the fluid is administered indirectly to the ocular surface by a delivery agent that is topically administered to the ocular surface or other part of the eye, preferably wherein the agent is a particle that is coated with and/or secretes the fluid on to the ocular surface.

11. The fluid for use according to any one of claims 1 to 10, wherein the fluid further comprises a preservative.

12. The fluid for use according to any one of claims 1 to 10, wherein the fluid is preservative-free.

13. The fluid for use according to claim 1, wherein the fluid further comprises an additional glycosaminoglycan (GAG), an electrolyte (such as sodium chloride), a buffer (such as phosphate buffer), or a combination of two or more of the foregoing.

Europäisches
Patentamt

European
Patent Office

Office européen
des brevets

# EUROPEAN SEARCH REPORT

Application Number

EP 21 16 7346

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Krzoska et al: "Effectiveness of Comfort Shield MDS results of a Clinical Investigation at Beuth Hocjschule für Tecknik, berlin.", Galifa Augenblick, July 2016 (2016-07), XP002777748, St Gallen CH Retrieved from the Internet: URL:https://www.icom-medical.de/upload/155 63389-Krzoska-Wirksamkeit-von-Comfort-Shie ld-Beuth-Studie-GALIFA-AUGENBLICK-2016-EN. pdf [retrieved on 2016-07] * Discussion. * | 1-13 | INV. A61K31/728 A61P27/04 |
| A | WO 2008/126803 A1 (SHISEIDO CO LTD [JP]; UEDA OSAMU [JP]; MATSUI RAKAN [JP]; OBARA TAKEO) 23 October 2008 (2008-10-23) * claims 1-3 * | 1-13 | |
| A | RAH ET AL: "A review of hyaluronan and its ophthalmic applications", OPTOMETRY - JOURNAL OF THE AMERICAN OPTOMETRIC ASSOCIATION, ELSEVIER, NL, vol. 82, no. 1, 1 January 2011 (2011-01-01), pages 38-43, XP027573889, ISSN: 1529-1839 [retrieved on 2010-12-23] * Last Paragraph * | 1-13 | TECHNICAL FIELDS SEARCHED (IPC) A61K |

The present search report has been drawn up for all claims

| Place of search | Date of completion of the search | Examiner |
|---|---|---|
| Munich | 20 July 2021 | Cattell, James |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 3 871 682 A1

## ANNEX TO THE EUROPEAN SEARCH REPORT
## ON EUROPEAN PATENT APPLICATION NO.

EP 21 16 7346

This annex lists the patent family members relating to the patent documents cited in the above-mentioned European search report.
The members are as contained in the European Patent Office EDP file on
The European Patent Office is in no way liable for these particulars which are merely given for the purpose of information.

20-07-2021

| Patent document cited in search report | Publication date | Patent family member(s) | Publication date |
|---|---|---|---|
| WO 2008126803 A1 | 23-10-2008 | JP 2008255061 A<br>WO 2008126803 A1 | 23-10-2008<br>23-10-2008 |

EPO FORM P0459

For more details about this annex : see Official Journal of the European Patent Office, No. 12/82

24

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62516911
- US 62408559
- US 5323907 A

- US 5052558 A
- US 5033252 A

### Non-patent literature cited in the description

- **NECAS J et al.** Hyaluronic acid (hyaluronan): a review. *Veterinarni Medicina,* 2008, vol. 53 (8), 397-411
- **STERN R et al.** Hyaluronan fragments: An information-rich system. *European Journal of Cell Biology,* 2006, vol. 85, 699-715
- **CYPHERT JM et al.** Size Matters: Molecular Weight Specificity of Hyaluronan Effects in Cell Biology. *International Journal of Cell Biology,* 2015
- **HIGASHIDE T ; K SUGIYAMA.** Use of viscoelastic substance in ophthalmic surgery- focus on sodium hyaluronate. *Clinical Ophthalmology,* 2008, vol. 2 (1), 21-30
- **POLACK FM ; MT MCNIECE.** *The treatment of dry eyes with Na hyaluronate (Healon) - preliminary report,* 1982, vol. 1 (2), 133-136
- **MULLER-LIERHEIM WGK.** Tranenersatzlosungen, Neues über Hyaluronsaure. *Aktuelle Kontaktologie,* April 2015, 17-19
- **TODA I et al.** Visual performance after reduced blinking in eyes with soft contact lenses or after LASIK. *J Refract. Surg.,* January 2009, vol. 25 (1), 69-73
- **REA MS ; MJ QUELLETTE.** Relative visual performance: A basis for application. *Lighting Res. Technol.,* 1991, vol. 23 (3), 135-144
- **BELMONTE C et al.** TFOS DEWS II pain and sensation report. *The Ocular Surface,* vol. 15, 404-437
- **TARNUZZER RW ; SCHULTZ GS.** Biochemical analysis of acute and chronic wound environments. *Wound Repair Regen.,* July 1996, vol. 4 (3), 321-325

- **PFLUGFELDER SC et al.** Epithelial-immune cell interaction in dry eye. *Cornea,* 2008, vol. 27 (1), 9-11
- **NAKAO et al.** *Am J Pathol.,* 2007, vol. 171 (3), 1058-65
- **KYMIONIS GD et al.** Treatment of chronic dry eye: treatment of cyclosporine. *Clin Ophthalmol.,* December 2008, vol. 2 (4), 829-836
- **SCHULTZ C.** Safety and efficacy of cyclosporine in the treatment of chronic dry eye. *Ophthalmol Eye Dis.,* 2014, vol. 6, 37-42
- **NOBLE PW.** Hyaluronan and its catabolic products in tissue injury and repair. *Matrix Biology,* 2002, vol. 21, 25-29
- **COWMAN MK et al.** *Carbohydrate Polymers,* 2000, vol. 41, 229-235
- **TAKIGAMI S et al.** *Carbohydrate Polymers,* 1993, vol. 22, 153-160
- Hyaluronan, its cross-linked derivative-Hylan-and their medical applications. **BALAZS EA et al.** Cellulosics Utilization: Research and Rewards in Cellulosics, Proceedings of Nisshinbo International Conference on Cellulosics Utilization in the Near Future. Elsevier Applied Science, 1989, 233-241
- **KOEHLER L et al.** *Scientific Reports,* 2017, vol. 7
- **PAVAN M et al.** *Carbohydr Polym,* 2013, vol. 97 (2), 321-326
- **CRAIG JP et al.** TFOS DEWS II Definition and Classification Report. *The Ocular Surface,* 2017, vol. 15, 276-283
- **BERGE S. M. et al.** Pharmaceutical Salts. *Journal of Pharmaceutical Science,* 1997, vol. 66, 1-19